# EUROPÄISCHE PATENTSCHRIFT

(11) **EP 1 066 293 B1**
(45) Veröffentlichungstag und Bekanntmachung des Hinweises auf die Patenterteilung: **17.07.2002**
(21) Anmeldenummer: 99914561.8
(22) Anmeldetag: 31.03.1999
(51) Int. Cl.: C07D 487/22, A61K 31/40

(54) **PORPHYRINE UND IHRE VERWENDUNG ALS PHOTOSENSITIZER**
NEW PORPHYRINS AND THEIR USE
NOUVELLES PORPHYRINES ET LEUR UTILISATION

(30) Priorität: 31.03.1998 DE 19814405
(43) Veröffentlichungstag der Anmeldung: 10.01.2001
(73) Patentinhaber: Schastak, Astrid, 04509 Zschortau (DE)
(72) Erfinder: SCHASTAK, Stanislaw, D-04509 Zschortau (DE); SHULGA, Alexander, Minsk, 220013 (BY); BERR, Frieder, D-04109 Leipzig (DE); WIEDEMANN, Peter, D-04229 Leipzig (DE)
(74) Vertreter: Freiherr von Wittgenstein, Arved, Dr.
(86) Internationale Anmeldenummer: EP9902228
(87) Internationale Veröffentlichungsnummer: WO9950269

(56) Entgegenhaltungen:
- EP-A- 0 337 601
- WO-A-96/13504
- WO-A-97/20846
- WO-A-97/32885
- DE-A- 19 627 164

## Beschreibung

Photosensitizer stellen chemische lichtempfindliche Verbindungen dar, die nach der Absorption des Lichtquantes eine photochemische Reaktion eingehen. In biologischer Umgebung werden sie von malignen als auch von einigen prämalignen Zellen infolge der metabolischen Prozesse in einer höheren Konzentration und auch für längere Zeit als in gesunden Zellen akkumuliert. Die Aktivierung des Photosensitizers erfolgt durch monochromatisches Laserlicht entsprechender Wellenlänge und ausreichender Intensität. Durch Lichtabsorption wird der Photosensitizer zuerst in einen relativ kurzlebigen Singulettzustand angeregt, der danach in einen stabileren Triplettzustand übergeht. Dieser angeregte Zustand kann zwei unterschiedlichen Reaktionen unterliegen.

Er kann direkt mit einem Substrat reagieren und Radikale bilden, die nach Reaktion mit dem Sauerstoff Peroxide, Hydroxylradikale, Superoxidanionradikale und andere Produkte bilden (Typ I-Reaktion), oder aber seine Energie auf Sauerstoff im Grundzustand übertragen und zur Bildung von Singulett-Sauerstoff ¹O₂ führen (Typ II-Reaktion). Für die meisten Photosensitizer wird dabei eine Wirkung über eine Typ II-Reaktion beschrieben. Der Singulett-Sauerstoff ist hochreaktiv und kann durch die Aufhebung des Spinnverbots leicht mit Biomolekülen oxidativ reagieren (Henderson, B.W. and Dougherty, T.J., How does photodynamic therapy work?, Photochem. Photobiol., 1992, 55, 145-157).

Spezifische Orte oder Typen der Zerstörung von Zellen durch die PDT sind bisher nicht exakt bekannt. Abhängig von der Art des jeweiligen Photosensitizers und dessen Ladung reichert sich dieser besonders an Zellmembranen, in Mitochondrien oder Lysosomen an. Es kommt dort zu einer Schädigung von Membranen durch Photooxidation von ungesättigten Fettsäuren, Lipidperoxidation und Protein-Crosslinking (Gomer C.J., Rucker N., Ferrario A., Wong S., Properties and applications of photodynamic therapy, Radiat. Res., 1989, 120, 1-18).

Diskutiert werden auch die Hemmung von bestimmten membranständigen Enzymen (Modica-Napoloitano J.S., Joxal J.L., Ara G., Oseroff A.R., Aprille J.R. Mitochrondial toxicity of cationic photosensitizers for phototherapy. Cancer Res., 1990, 50, 7876-7881), eine Veränderung der intrazellulären Ca²⁺-Ionenkonzentration (Hubmer A., Hermann A., Überriegler K., Krammer B., Role of calcium in photodynamically induced cell damage of human fibroblasts, Photochem. Photobiol., 1996, 64, 211-215) und die Induktion von Apoptose (Luo Y., Chang C.K., Kessel D., Rapid initiation of apoptosis by photodynamic therapy, Photochem. Photobiol., 1996, 63, 528-534).

In der Medizin wird diese Prozedur als photodynamische Therapie (PDT) bezeichnet und ist eine der vielversprechendsten Behandlungsmethoden in der Onkologie. Die PDT wird als Methode der Wahl immer dann eingesetzt, wenn die Patienten entweder zu alt oder zu schwach sind, um chemotherapeutische, chirurgische bzw. radiologische Eingriffe zu verkraften, oder aber, wenn diese bereits versagt haben. Die PDT kann auch gemeinsam und zusätzlich zu anderen Tumortherapien und mehrmals an einem Patienten eingesetzt werden, wobei die Wirkung durch die Synergieeffekte noch gesteigert wird. Die Bestrahlungszeiten betragen einige Minuten, wodurch der Einsatz von cw-Lasern und der Wellenleiter zum Lichttransport erforderlich werden.

Obwohl die PDT als eine Therapiemethode bereits 1900 von Raab (Raab O., Über die Wirkung fluoreszierender Stoffe auf Infusoria, Z. Biol., 1900, 39, 524-526) vorgeschlagen wurde, konnten erst in den 60er Jahren substantielle Fortschritte gemacht werden, als gezeigt wurde, daß Hämatoporphyrinderivate (HPD) selektiv in Tumorgewebe angereichert werden können (Lipson R., Baldes E., Olson A., The use of a derivative of hematoporphyrin in tumor detection, J. Natn. Cancer Inst., 1961, 267, 1-8). Seitdem wurden umfangreiche Untersuchungen mit HPD durchgeführt (Kessel D. (ed.), Photodynamic Therapy of Neoplastic Disease, Vols. I and II. CRC Press, Boston 1990; Moan J., Porphyrin photosensitization and phototherapy, Photochem. Photobiol., 1986, 43, 681-690; Pass H.I., Photodynamic therapy in oncology: Mechanisms and clinical use, J. Natl. Cancer Inst., 1993, 85, 443-456). Die Behandlung mit dem Photofrin® ist zur Zeit in einigen Ländern für einige Indikationen klinisch zugelassen.

Aufgrund der vielversprechenden klinischen Resultate, die man mit dem Photofrin® erhalten hat, und bedingt durch verschiedene Nachteile dieser Substanz (geringe Absorption im Bereich von 700 bis 900 nm, d.h. in einem Bereich, wo die Eigenabsorption des Gewebes minimal ist, chemische Heterogenität, hohe und langanhaltende Phototoxizität bei Tageslicht u.a.) werden zunehmend sogenannte Photosensitizer der zweiten und der dritten Generation synthetisiert und getestet. Diese Gruppe umfaßt zahlreiche Stoffklassen wie z.B. Anthrachinone, Anthrapyrazole, Perylenchinone, Xanthene, Cyanine, Acridine, Phenoxazine und Phenothiazine (Diwu Z.J., Lown J.W. Phototherapeutic potential of alternative photosensitizers to porphyrins, Pharmac. Ther., 1994, 63, 1-35). Die Photosensitizer der dritten Generation werden nach folgenden Kriterien ausgewählt (Gomer C.J., Rucker N., Ferrario A., Wong S. Properties and applications of photodynamic therapy, Radiat. Res., 1989, 120, 1-18) :
- chemische Reinheit
- Wasserlöslichkeit
- minimale Dunkeltoxizität
- signifikante Absorption bei Wellenlängen über 700 nm
- hohe Ausbeuten an Singulett-Sauerstoff
- vorwiegende Lokalisation im pathologischen Gewebe (z. B. Tumor)
- schnelle Ausscheidung aus normalem Gewebe.

Zur Zeit werden sehr intensiv die Forschungsarbeiten zur Synthese von Bacteriochlorin-Derivaten geführt. Diese sollen die oben gestellten Auswahlkriterien weitestgehend erfüllen (Dougherty T.J. et al. WO 90/12573; Skalkos D. et al. WO 94/00118; Pandey R.K. et al. WO 95/32206; Dolphin D. et al. WO 96/13504; Pandey R.K. et al. WO 97/32885). Einige der neu synthetisierten Bakteriochlorinen weisen eine vernachlässigbare Dunkeltoxizität und eine hohe Tumorselektivität auf, sind teilweise wasserlöslich und besitzen starke Absorptionsbanden im Bereich zwischen 700 nm bis 810 nm im sogenannten "phototherapeutischen Fenster". Somit ermöglichen sie eine Behandlung von Gewebeschichten, die tiefer als 1 cm liegen (Pandey R., Kozyrev A., Potter W.R., Henderson B.W., Bellnier T.J., Dougherty T.J.: Long wavelength photosensitizers for photodynamic therapy, Photochem. Photobiol., 1996, 63, Abstracts of the 24th Annual Meeting of the American Soc. for Photobiology, TPM-E6).

Zu den Nachteilen der bekannten Bakteriochlorin-Derivaten gehören:
- kompliziertes und teures Syntheseverfahren, das meistens eine Reinigung des Ausgangsproduktes erforderlich macht,
- ihre schlechte Wasserlöslichkeit, die bei einer systemischen Applikation eine Auflösung in organischen Lösungsmitteln zur Folge hat und eine zusätzliche chemische Belastung des Organismus bedeutet,
- ihre negative bzw. neutrale Gesamtladung, wodurch die Aufnahme durch die Zellen erschwert wird, da sie normalerweise negativ geladen sind,
- chemische Instabilität des Produktes.

Aufgabe der vorliegenden Erfindung ist es, die genannten Nachteile zu vermeiden und Photosensitizer zur Verfügung zu stellen, deren chemische und physikalische Eigenschaften eine technisch und wirtschaftlich sinnvolle Nutzung erlauben.

Gelöst wird diese Aufgabe durch neue Porphyrine der folgenden allgemeinen Formel: 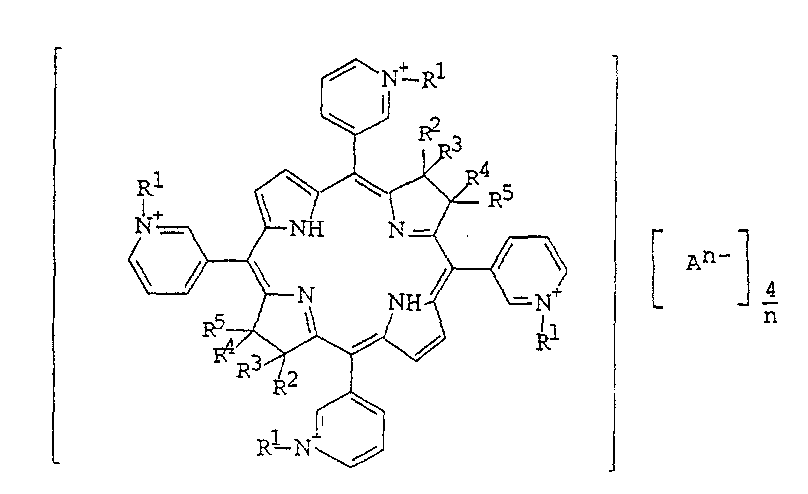 worin bedeuten:
- R¹: C₁ bis C₆-Alkyl oder Aryl,
- R² bis R⁵: unabhängig voneinander H, OH, C₁ bis C₆-Alkyl, C₁ bis C₆-Alkylen oder OR⁶, wobei R⁶ die Bedeutung von C₁ bis C₆-Alkyl oder Aryl hat,
- Aⁿ⁻: ein Anion und
- n: 1 oder 2.

Das ein- oder zweiwertige Anion wird vorzugsweise ausgewählt aus der Gruppe bestehend aus Cl⁻, Br⁻, J⁻, 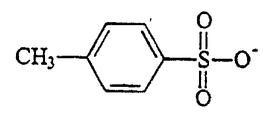 SO₄²⁻.

Bei einer besonders bevorzugten Ausführungsform bedeuten R¹ eine C₁ bis C₆-Alkylgruppe, d.h. Methyl, Ethyl, Propyl, Butyl, Pentyl, Hexyl, insbesondere Methyl, die Reste R² bis R⁵ H und das Anion Aⁿ⁻

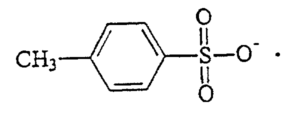

Die neuen Porphyrine können auf einfache Weise hergestellt werden. Als Ausgangsprodukte dienen hierbei Porphyrine der allgemeinen Strukturformel: 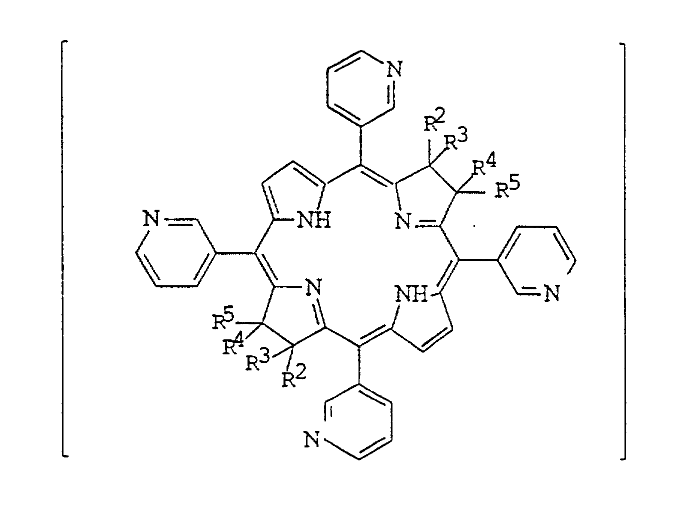 wobei R² bis R⁵ die obige Bedeutung haben. Diese Produkte sind im Handel erhältlich oder können auf einfache Weise aus der handelsüblichen Verbindung, bei der R² bis R⁵ die Bedeutung von H haben, synthetisiert werden.

Die Ausgangsverbindungen werden mit einer Verbindung der allgemeinen Formel R¹A umgesetzt, in welcher R¹ und A die obige Bedeutung haben.

Bei einer bevorzugten Ausführungsform des Verfahrens wird als Verbindung R¹A Methyltosylat, 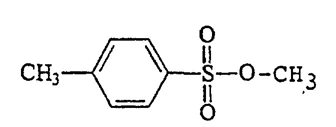 verwendet.

Die erfindungsgemäßen neuen Verbindungen gehören zur Klasse der Tetrakis-pyridyl-porphyrin-derivate und werden bevorzugt als Photosensitizer in der Medizin, Landwirtschaft und Industrie bei photodynamischen Verfahren eingesetzt. Das wesentliche dabei ist der Einsatz des Pyridyl-Derivats des Porphins als aktive photochemische Substanz. Besonders vorteilhaft ist die große Wasserlöslichkeit und chemische Stabilität der neuen Verbindungen.

Des weiteren können die neuen Verbindungen mittels Metallkomplexen modifiziert werden. In diesem Zusammenhang ergeben sich die Möglichkeiten des Transports von ausgewählten Metallen (z.B. seltene Erden usw.) in Tumorzellen, mit dem Ziel sie dort zu akkumulieren. Dadurch können neue Absorptionslinien im nahen Infrarot-Bereich erzeugt werden, die sowohl für die Diagnose als auch für die Therapie interessant sind. Bei der Verwendung von Lanthaniden besteht außerdem die Möglichkeit der Anregung des so gebildeten Sensitizer-Komplexes mittels Röntgen-Strahlung. Danach wird diese Anregungsenergie mittels strahlungsloser bzw. strahlende Energietransfer-Prozesse auf den molekularen Sauerstoff übertragen, so daß der Singulett-Sauerstoff als reaktive Spezies gebildet wird.

Die neuen erfindungsgemäßen Verbindungen eignen sich auch für die Behandlung schwarzer Melanome.

Spezielle Ausführungsbeispiele der Erfindung sind nachfolgend wiedergegeben.

### Herstellungsbeispiel

### Synthese von 5,10, 15,20-Tetrakis-(1-alkyl-3-pyridyl)-21H,23H-7,8,17,18-tetrahydroporphyrin-tetra-p-toluolsulfonat

150 mg Tetrakis-(3-pyridyl)-21H,23H-7,8,17,18-tetrahydroporphyrins werden in 15 ml Nitromethan aufgelöst. Danach werden 350 mg Methyltosilat dazugegeben und das Ganze 2 Stunden lang unter Stickstoff-Atmosphäre gekocht.

Nach 2 Stunden werden 120 mg Methyltosilat zugegeben und das Kochen noch 3 Stunden lang fortgesetzt. Nach dem Reaktionsende wird die Lösung eingeengt und bei Zimmertemperatur 12 Stunden lang stehengelassen. Das kristalline Sediment wird filtriert und mit einer Benzol-Nitromethan-Lösung (1:1) gewaschen.

Die Ausbeute beträgt 238 mg (100 %-igen) 5,10,15,20-Tetrakis-(1-alkyl-pyridyl)-21H,23H-7,8,17,18-Tetrahydroporphyrinstetra-p-toluolsulfonats (THPTPS). Das sind 72 % der Masse der Ausgangsstoffe.
Chemische Formel: C₇₂H₇₀N₈O₁₂S₄.
Molekulargewicht: MG = 1367,66.

### Anwendungsbeispiel

Der Zeitpunkt der maximalen Akkumulation des Sensitizers in den Zellen als eine Funktion der Inkubationszeit wird mit Hilfe eines Fluoreszenz-Spektrometers FluoroMax-2 (Jobbin YVON-Spex Instruments S.A. Inc.) bestimmt. Die Zellen des Aderhautmelanom (Schastak S.I., Enzmann V., Jüngel A., Zhavrid E.A., Voropai E.S., Alexandrova E.N., Samtsov M.P., Uugovssky A.P. und P. Wiedemann, Erste Ergebnisse zur PDT des Aderhautmelanoms ex vivo mittels neuer im NIR-Bereich absorbierenden Photosensitizer, Lasermedizin 1997, 13: 50-54) sowie die Zellen einer hochdifferenzierten Gallenblasenkarzinom-Zellinie (Wittier) (Purdum, P.P., Cultrued human gallbladder epithelia, A carcinoma-derived model, Lab. Invest., 1993, 68: 345-353) und einer Gallengangsadenokarzinom-Zellinie (Charies), hochgradig undifferenziert (etabliert von Prof. P. Hylemon, Virginia Commonwealth Univ., Richmond, VA, 1996) mit einer Zellzahl von 1,2x10⁶ Zell/ml werden mit dem Sensitizer THPTPS in einer Konzentration von 0,4 mg/ml bzw. 0,2 mg/ml (LD₁₀) 1, 6, 12 und 24 Stunden im Dunkeln inkubiert. Danach werden sie dreimal im PBS gewaschen, abtrypsiniert, mit dem Ultraschall aufgeschlossen und mit 5000 U/min 20 Minuten zentrifugiert. Die Menge des Photosensitizers im Überstand entspricht dabei genau derjenigen Menge, die in den Zellen akkumuliert ist. Durch die Messung der Intensität im Maximum der Emissionsbande des THPTPS (λₒ = 775 nm) nach unterschiedlichen Inkubationszeiten erhält man die Abhängigkeit der Fluoreszenzintensität von der Inkubationszeit, die den Zeitpunkt der maximalen Aufnahme des Sensitizers in die Zellen aufzeigt.

Für die Untersuchung der photodynamischen Wirkung in vitro wurden die o.g. Zellinien in einer Zellzahl von 2 x 10⁵ Z/ml im DMEM + 10 % FKS kultiviert und mit dem Photosensitizer THPTPS in einer Konzentration von 0,2, 0,4, 0,8 mg/ml (Wittier) bzw. 0,4, 0,8, 1,6 mg/ml 12 Stunden inkubiert. Danach bestrahlte man sie mit dem Licht eines Ti:Sa-Lasers mit einer Wellenlänge von 771 nm und einer Dosis von 15 J/cm². Die Auswertung der Bestrahlungsergebnisse mittels MTT-Tests (3-[4,5-Dimethylthiazol-2-yl] -2,5-diphenyltetrazolium-bromid) zeigte, daß bei der hochdifferenzierten Gallenblasenkarzinom-Zellinie ca. 88 % und bei der hochgradig undifferenzierten Gallengangsadenokarzinom-Zellinie ca. 84 % der Zellen tot sind. Etwa 88 % der Aderhautmelanom-Zellen konnten durch die Bestrahlung getötet werden.

## Patentansprüche

1. Verbindungen der allgemeinen Formel: 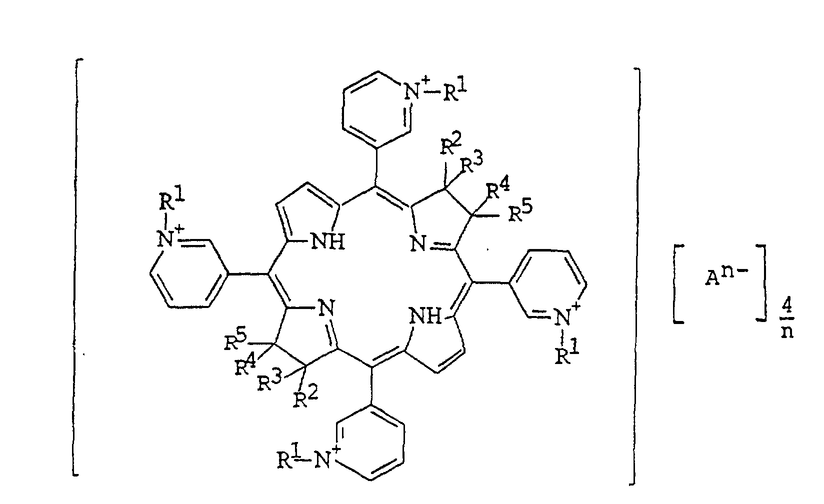 in welcher bedeuten:
R¹ C₁ bis C₆-Alkyl oder Aryl,
R² bis R⁵ unabhängig voneinander H, OH, C₁ bis C₆-Alkyl, C₁ bis C₆-Alkylen oder OR⁶, wobei R⁶ die Bedeutung von C₁ bis C₆-Alkyl oder Aryl hat,
Aⁿ⁻ ein Anion und
n 1 oder 2.

2. Verbindungen nach Anspruch 1, **dadurch gekennzeichnet, daß** R¹ die Bedeutung von Methyl, Ethyl, Propyl, Butyl, Pentyl oder Hexyl, inbesondere Methyl hat.

3. Verbindungen nach Anspruch 1 oder 2, **dadurch gekennzeichnet, daß** R² bis R⁵ jeweils die Bedeutung von H haben.

4. Verbindungen nach Anspruch 1 bis 3, **dadurch gekennzeichnet, daß** Aⁿ⁻ ausgewählt ist aus Cl⁻, Br⁻, J⁻,

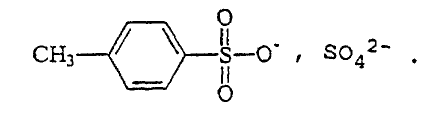

5. Verbindung nach den Ansprüchen 1 bis 4, **dadurch gekennzeichnet, daß** R¹ CH₃, R² bis R⁵ jeweils H und Aⁿ⁻ 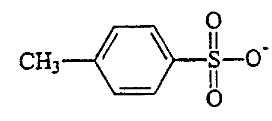 bedeuten.

6. Verwendung der Verbindungen nach den Ansprüchen 1 bis 5 als Photosensitizer.

## Claims

1. Compounds of the general formula: 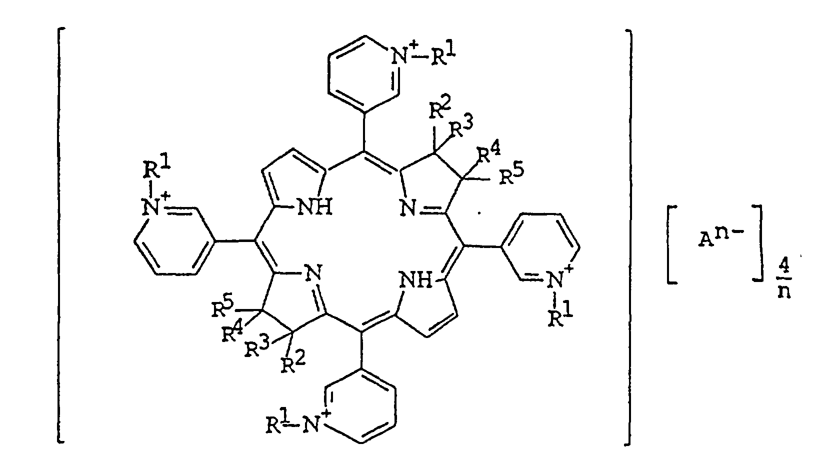 in which:
R¹ is C₁ to C₆ alkyl or aryl,
R² to R⁵ independently of each other are H, OH, C₁ to C₆ alkyl, C₁ to C₆ alkylene or OR⁶, R⁶ being C₁ to C₆ alkyl or aryl,
Aⁿ⁻ is an anion and
n is 1 or 2.

2. Compounds according to claim 1, **characterized in that** R¹ is methyl, ethyl, propyl, butyl, pentyl or hexyl, in particular methyl.

3. Compounds according to claim 1 or 2, **characterized in that** R² to R⁵ are each H.

4. Compounds according to claims 1 to 3, **characterized in that** Aⁿ⁻ is selected from Cl⁻, Br⁻, J⁻,

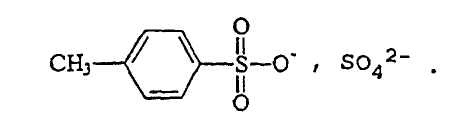

5. Compound according to claims 1 to 4, **characterized in that** R¹ is CH₃, R² to R⁵ are each H and Aⁿ⁻ is

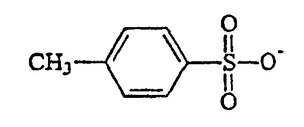

6. Use of the compounds according to claims 1 to 5 as photosensitizers.

## Revendications

1. Composés selon la formule générale : 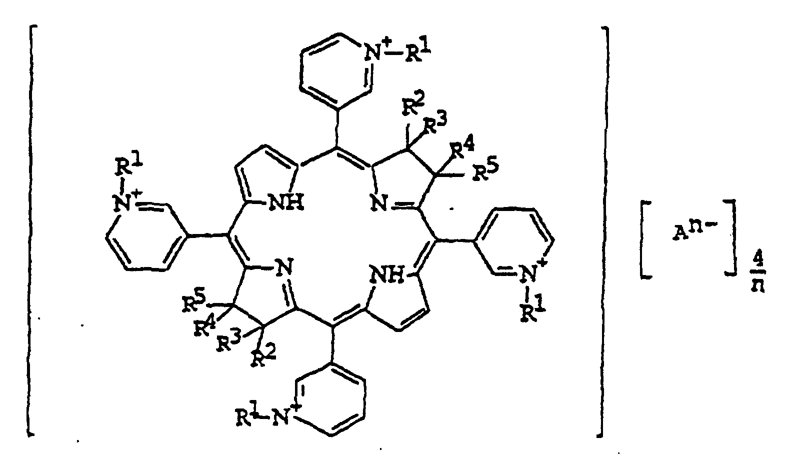 dans laquelle :
R¹ est un groupe alkyle C₁ à C₆ ou un groupe aryle,
R² à R⁵ sont indépendamment H, OH, un groupe alkyle C₁ à C₆, un groupe alkylène C₁ à C₆ ou OR⁶, où R⁶ est un groupe alkyle C₁ à C₆ ou un groupe aryle,
Aⁿ⁻ est un anion et
n est 1 ou 2.

2. Composés selon la revendication 1, **caractérisés en ce que** R¹ est un groupe méthyle, éthyle, propyle, butyle, pentyle ou hexyle, en particulier un groupe méthyle.

3. Conposés selon la revendication 1 ou 2, **caractérisés en ce que** R² à R⁵ sont chacun H.

4. Composés selon une des revendications 1 à 3, **caractérisés en ce que** Aⁿ⁻ est choisi parmi Cl⁻, Br⁻, J⁻,

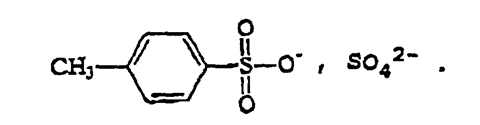

5. Composé selon une des revendications 1 à 4, **caractérisé en ce que** R¹ est CH₃, R² à R⁵ sont chacun H et Aⁿ⁻ est

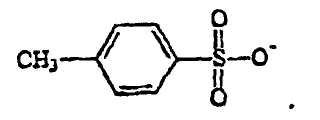

6. Utilisation des composés selon les revendications 1 à 5 comme photosensibilisants.
